# EUROPEAN PATENT APPLICATION

(11) **EP 4 537 818 A1**
(43) Date of publication of application: **16.04.2025**
(21) Application number: 23824211.9
(22) Date of filing: 13.06.2023
(51) Int. Cl.: A61K 9/51, A61K 9/127, B82Y 5/00, B82Y 40/00

(54) **METHOD FOR SYNTHESIZING HYBRID NANOPARTICLES CONTAINING APOLIPOPROTEINS**

(30) Priority: 13.06.2022 KR 20220071495
(71) Applicant: Mepsgen Co., Ltd., Seoul 05836 (KR)
(72) Inventor: JEON, Mi Yeon, Seoul 05667 (KR); LEE, Woo Seung, Seoul 05667 (KR); KIM, Se Yeon, Seoul 05518 (KR); KIM, Yong Tae, Seoul 05667 (KR)
(74) Representative: WP Thompson
(86) International application number: PCT/KR2023/008146
(87) International publication number: WO 2023/243997

(57) **Abstract**

The present invention relates to a method for synthesizing hybrid nanoparticles comprising apolipoproteins. Specifically, the present invention relates to a method for synthesizing hybrid nanoparticles by using a swirling microvortex device, wherein the hybrid nanoparticles may include proteins that include apolipoproteins and have various functionalities. A production method according to the present invention can be used to produce small, uniform, stable nanoparticles having various physiological activities.

## Description

### Technical Field

The present invention relates to a method for synthesizing hybrid nanoparticles comprising apolipoproteins. Specifically, the present invention relates to a method for synthesizing hybrid nanoparticles by using a swirling microvortex device, wherein the hybrid nanoparticles may include proteins that include apolipoproteins and have various functionalities. A production method according to the present invention can be used to produce small, uniform, stable nanoparticles having various physiological activities.

### Background Art

Nanoparticles refer to particles having a size of less than micrometer. Due to their size, they can move freely in the body and can be given various physical properties depending on the constituent materials, and are attracting attention as next-generation drug delivery vehicles.

Nanoparticles for drug delivery are composed of various components such as phospholipids and polymers, depending on the need, and each has different characteristics depending on the constituent materials. Nanoparticles made of inorganic materials are mainly used for diagnostic purposes, and polymer nanoparticles are mainly used as drug delivery vehicles for the purpose of controlling the release rate of drugs and controlling the circulation time in the body. Phospholipid nanoparticles are amphiphilic, easy to self-assemble, and have various molecular structures, so various types of phospholipid nanoparticles are being studied as drug delivery vehicles.

Recently, hybrid nanoparticles that combine the advantages of these nanoparticles are being studied. However, existing methods for producing nanoparticles mainly consist of non-standardized multi-step processes such as nanoprecipitation and emulsification-based solvent evaporation, so there are many difficulties in mass-producing hybrid nanoparticles with uniform sizes using existing methods for producing nanoparticles. In particular, it is difficult to synthesize polymers such as functional proteins in nanoparticles at once due to the complexity of the synthesis, and if produced through many processes, the uniformity and stability are lost, making mass production difficult.

The present inventors studied a synthesis method capable of incorporating a functional protein in a polymer-lipid hybrid nanoparticle (PHNP) using PLGA (poly(lactic-co-glycolic acid)), a polymer that is biodegradable and biocompatible while protecting drugs from degradation, thereby completing the present invention.

### Prior Art Document

### Non-Patent Document

(Non-Patent Document 1) Toth et al., Robust manufacturing of lipid-polymer nanoparticles through feedback control of parallelized swirling microvortices. 2017, Lab. Chip., 17.16: 2805-2813.

### Detailed Description of Invention

### Technical Problem

An object of the present invention is to provide a method for producing a hybrid nanoparticle comprising a mixture of hydrophilic and hydrophobic substances by mixing a phospholipid and a polymer under a swirling microvortex.

Another object of the present invention is to provide a method for producing a hybrid nanoparticle comprising a protein, wherein the method comprises a step of adding a protein to the hybrid nanoparticle by colliding the protein with the hybrid nanoparticle under a swirling microvortex.

In addition, another object of the present invention is to provide a hybrid nanoparticle comprising a protein produced by the above method.

### Solution to Problem

The present invention provides a method for producing a hybrid nanoparticle comprising a protein, wherein the method comprises: a step of injecting a hybrid nanoparticle into the first inlet and injecting a protein into the second inlet in a swirling microvortex device comprising a first inlet, a second inlet, and an outlet; and a step of adding the protein to the hybrid nanoparticle under a swirling microvortex.

In one embodiment, the Reynolds flow rate in the swirling microvortex device may be 50 to 300.

In one embodiment, the protein may be an apolipoprotein or a polymer having amphiphilic properties.

In one embodiment, the apolipoprotein may be at least one selected from the group consisting of apolipoproteins A1, A2, E2, E3, J, and M.

In one embodiment, the synthetic mixing weight ratio of the hybrid nanoparticle and the apolipoprotein may be 20:1 to 0.5:1, and preferably, may be 20:1 to 2:1.

In one embodiment, the production method may further comprise a step of recovering a nanoparticle comprising a protein from the outlet.

In one embodiment, the hybrid nanoparticle may be produced by a production method comprising: a step of injecting a phospholipid into the first inlet and injecting a polymer into the second inlet in the swirling microvortex device; and a step of mixing the phospholipid and the polymer under a swirling microvortex.

The phospholipid may be at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-distearoyl-sn-glycero-3-phosphocholine (DAPC), 1,2-diarachidoyl-sn-glycero-3-phosphocholine (DBPC), 1,2-diicosanoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), distearoylphosphatidylethanolamine-polyethylene glycol (DSPE-PEG), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-aminopropyl)amino]butylcarboxamido)ethyl]-3,4-di[oleyloxy]-benzamide) (VL-5), dioctadecylamidoglycylspermine 4-trifluoroacetic acid (DOGS), 3β-[N-(N',N'-dimethylaminoethane)-carbamoyl]cholesterol (DC-Chol), 1,2-di-O-octadecenyl-3-trimethylammonium propane (DOTMA), 1,2-dioleyl-3-trimethylammonium-propane (DOTAP), (1,2-dioleyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), and D-Lin-MC3-DMA (MC3, DLin-MC3-DMA), DLin-KC2-DMA, DLin-DMA, and preferably, may be DPPC and DSPE-PEG, and more preferably, may be DPPC and DSPE-PEG2000 to 5000.

In one embodiment, the ratio of DPPC and DSPE-PEG2000 may be 2.3:1 to 1:1.

In one embodiment, the polymer may be PLGA (poly(lactic-co-glycolic acid)).

In one embodiment, the hybrid nanoparticle may be a polymer-lipid hybrid nanoparticle (PHNP).

The present invention provides a hybrid nanoparticle comprising a protein produced by the above production method.

The present invention provides a method for producing a hybrid nanoparticle comprising a protein, wherein the method comprises: a first step for producing a hybrid nanoparticle, wherein the first step comprises a step of injecting a phospholipid into the first inlet and injecting a polymer into the second inlet in a swirling microvortex device comprising a first inlet, a second inlet, and an outlet; and a step of mixing the phospholipid and the polymer under a swirling microvortex; a second step for producing a hybrid nanoparticle comprising a protein, wherein the second step comprises a step of injecting the obtained hybrid nanoparticle into the first inlet and injecting a protein into the second inlet.

In one embodiment, the protein may be an apolipoprotein or a polymer having amphiphilic properties.

In one embodiment, the synthetic mixing weight ratio of the hybrid nanoparticle and the apolipoprotein may be 20: 1 to 2:1.

In one embodiment, the polymer may be PLGA (poly(lactic-co-glycolic acid)).

In one embodiment, the hybrid nanoparticle may be a polymer-lipid hybrid nanoparticle (PHNP).

### Effects of Invention

The polymer-lipid hybrid nanoparticle (PHNP) produced by the production method according to the present invention comprises a hydrophilic substance and a hydrophobic substance at the same time, and has a form in which the center of the nanoparticle made of the polymer is surrounded by a hydrophobic substance, and can effectively incorporate and deliver a high concentration of hydrophobic drug inside.

In addition, the hybrid nanoparticle of the present invention can be used as a material capable of delivering multiple drugs by utilizing the surface, middle shell portion, and core portion, respectively. For example, the hybrid nanoparticle of the present invention can be used as an effective delivery vehicle for anionic drugs such as genes.

In addition, a polymer such as PEG located on the surface of the hybrid nanoparticle can prevent foreign substances from being adsorbed to the particle surface or removed by immune cells in the human body, thereby enabling safer drug delivery. Therefore, it can be used for intracellular drug delivery, etc., to help with efficient treatment.

### Brief Description of Drawings

Figure 1 is a schematic diagram of the synthesis process of hybrid nanoparticles (PHNP-E3/A1) comprising both ApoE3 and ApoA1 and the shape of PHNP-E3/A1.
Figure 2 is a graph comparing the size of hybrid nanoparticles (PHNP-2000) according to the synthetic mixing weight ratio of DSPE-PEG2000 to DPPC.
Figure 3 is a TEM photograph of hybrid nanoparticles (PHNP-2000) according to the synthetic mixing weight ratio of DSPE-PEG2000 to DPPC of Figure 2.
Figure 4 is a graph comparing the size of hybrid nanoparticles (PHNP) according to the PEG molecular weight of DSPE-PEG.
Figure 5 is a TEM photograph of hybrid nanoparticles (PHNP) according to the PEG molecular weight of DSPE-PEG of Figure 4.
Figure 6 is a graph showing the stability of each nanoparticle by measuring the size of the hybrid nanoparticles (PHNP) synthesized in Figure 4 on the day of synthesis and 3 days after synthesis.
Figure 7 is a schematic diagram of the synthesis process of hybrid nanoparticles (PHNP-E3) comprising ApoE3 using a swirling microvortex device and the shape of PHNP-E3.
Figure 8 is a graph comparing the sizes of PHNP-PEG2000 and PHNP-E3.
Figure 9 is a TEM photograph of PHNP-E3.
Figure 10 is a graph comparing the size of PHNP-E3 synthesized under each Reynolds flow rate condition.
Figure 11 is a graph showing the yield amount of ApoE3 included in PHNP-E3.
Figure 12 is a schematic diagram of the synthesis process of hybrid nanoparticles (PHNP-A1) comprising ApoA1 and the shape of the hybrid nanoparticle (PHNP-A1).
Figure 13 is a graph comparing the sizes of PHNP-PEG2000 and PHNP-A1.
Figure 14 is a TEM photograph of PHNP-A1.
Figure 15 is a fluorescence photograph of hybrid nanoparticles (PHNP-PEG2000) labeled with rhodamine being delivered into HAECs cells.
Figure 16 is a fluorescence photograph of hybrid nanoparticles (PHNP-A1) labeled with rhodamine being delivered into HAECs cells.
Figure 17 is a graph comparing the results obtained by quantifying the fluorescence intensities of rhodamine in Figures 15 and 16.
Figure 18 is a graph comparing the size and polydispersity index (PDI) of PHNP-A1 (SMR) and PHNP-A1 (BT) synthesized with or without a swirling microvortex device.
Figure 19 is a graph comparing the yield amount of each ApoA1 using ELISA after synthesizing PHNP-A1 (SMR) and PHNP-A1 (BT) according to the synthesis weight ratio of PHNP to ApoA1.
Figure 20 is a graph comparing the yield rate of each ApoA1 using ELISA after synthesizing PHNP-A1 (SMR) and PHNP-A1 (BT) according to the synthesis weight ratio of PHNP to ApoA1.
Figure 21 is a fluorescence photograph showing the delivery of PHNP-A1 (SMR) and PHNP-A1 (BT) into iHBECs, and a graph comparing the results obtained by quantifying the fluorescence.
Figure 22 is a graph showing the size distribution of PHNP-E3/A1 synthesized according to each mixing weight of ApoE3 and ApoA1.
Figure 23 shows the size and PDI results on the day of synthesis, 1 day, 3 days, 7 days, and 14 days after synthesis, showing the stability of PHNP-E3/A1.
Figure 24 is a graph showing the fluorescence wavelength of PHNP-PEG2000, PHNP-E3, PHNP-A1, and PHNP-E3/A1.

### Best Mode for Carrying out the Invention

Hereinafter, with reference to the accompanying drawings, embodiments and examples of the present invention will be described in detail so that those of ordinary skill in the art to which the present invention belongs can easily practice the present invention. However, the present invention may be implemented in various forms and is not limited to the embodiments and examples described herein.

Throughout the present specification, when a certain part "includes" a certain component, it means that other components may be further included, rather than excluding other components, unless otherwise stated.

As used herein, the term "hybrid nanoparticle" refers to a nanomaterial formed by the coexistence of a hydrophilic substance and a hydrophobic substance, and preferably refers to a nanoparticle in which a polymer and a lipid are mixed (polymer-lipid hybrid nanoparticle; PHNP), and is also referred to as "PHNP".

As used herein, the term "PLGA" refers to poly(lactic-co-glycolic acid), which is a polymer that is biodegradable and biocompatible.

As used herein, the term "DPPC" refers to dipalmitoylphosphatidylcholine, which is a type of lipid.

As used herein, the term "DSPE-PEG" refers to a lipid in which polyethylene glycol (PEG) is bound to distearoylphosphatidylethanolamine, and preferably, the molecular weight of the polyethylene glycol may be 2000 to 5000.

As used herein, the term "polymer having amphiphilic properties" refers to a protein that has both hydrophobic and hydrophilic properties, and examples include glycophorin, rhodopsin, CD36 (cluster of differentiation 36), seipin, glucose permease, cytochrome c, cupredoxins, high potential iron protein, adrenodoxin reductase, flavoprotein, and the like.

As used herein, the term "phospholipid" may be specifically at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-distearoyl-sn-glycero-3-phosphocholine (DAPC), 1,2-diarachidoyl-sn-glycero-3-phosphocholine (DBPC), 1,2-diicosanoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), distearoylphosphatidylethanolamine-polyethylene glycol (DSPE-PEG), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-aminopropyl)amino]butylcarboxamido)ethyl]-3,4-di[oleyloxy]-benzamide) (VL-5), dioctadecylamidoglycylspermine 4-trifluoroacetic acid (DOGS), 3β-[N-(N',N'-dimethylaminoethane)-carbamoyl]cholesterol (DC-Chol), 1,2-di-O-octadecenyl-3-trimethylammonium propane (DOTMA), 1,2-dioleyl-3-trimethylammonium-propane (DOTAP), (1,2-dioleyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), and D-Lin-MC3-DMA (MC3, DLin-MC3-DMA), DLin-KC2-DMA, DLin-DMA, but is not limited thereto.

The term "apolipoprotein E" refers to a mammalian protein encoded by the APOE gene or a functional variant thereof. In a preferred embodiment, the apolipoprotein E is a human protein encoded by the human APOE gene on chromosome 19. The apolipoprotein E may be any one of the isoforms of the APOE gene product, such as apolipoprotein E2 ("APOE2"), apolipoprotein E3 ("APOE3"), and apolipoprotein E4 (APOE4). APOE is a polymorphism having three major alleles (epsilon 2, epsilon 3, and epsilon 4). Any of the alleles may be used in various embodiments of the present invention. A "functional variant" of the present invention refers to a variant of a mammalian protein encoded by an APOE gene that retains the same or similar biological function as the APOE gene product. In some cases, the functional variant comprises amino acid insertions, deletions, and/or substitutions compared to a protein encoded by a human APOE gene. In some cases, the functional variant is a fragment of a protein encoded by a human APOE gene.

In some embodiments, the apolipoprotein E3 is a protein disclosed in GenBank under the accession number ARQ79461.1 or a protein having at least 95% sequence identity thereto, preferably at least 98% or 99% sequence identity thereto.

In some embodiments, the apolipoprotein E in the hybrid nanoparticle is a recombinant protein produced by genetic engineering, or a synthetic protein produced by chemical synthesis.

The term "apolipoprotein A1" refers to a mammalian protein encoded by the APOA1 gene or a functional variant thereof. In a preferred embodiment, the apolipoprotein A1 is a human protein encoded by the human APOA1 gene located on chromosome 11. A "functional variant" thereof refers to a variant of a mammalian protein encoded by the APOA1 gene that retains the same or similar biological function as the APOA1 gene product. In some cases, the functional variant comprises amino acid insertions, deletions, and/or substitutions compared to the protein encoded by the human APOA1 gene. In some cases, the functional variant is a fragment of a protein encoded by the human APOA1 gene.

In some embodiments, the apolipoprotein A1 is a protein disclosed in GenBank under the accession number AAS68227.1 or a protein having at least 95% sequence identity thereto, preferably at least 98% or 99% sequence identity thereto.

In some embodiments, the apolipoprotein A1 in the hybrid nanoparticle is a recombinant protein produced by genetic engineering, or a synthetic protein produced by chemical synthesis.

As used herein, the term "apolipoprotein E" is meant to encompass a fragment and a functional variant thereof.

As used herein, the term "swirling microvortex" refers to a small fluid flowing in a rotational motion in a fluid flow.

As used herein, the term "microvortex device" refers to a device including a microchannel, etc., which is provided to allow a fluid to flow on a substrate made of various materials including plastic, glass, metal, or silicon comprising an organic polymer material.

Hereinafter, the present invention will be described in more detail through the examples, but the following examples are for the purpose of explanation only and are not intended to limit the scope of the present invention.

### [Example 1]

### Method for synthesizing hybrid nanoparticles comprising apolipoproteins using swirling microvortex device

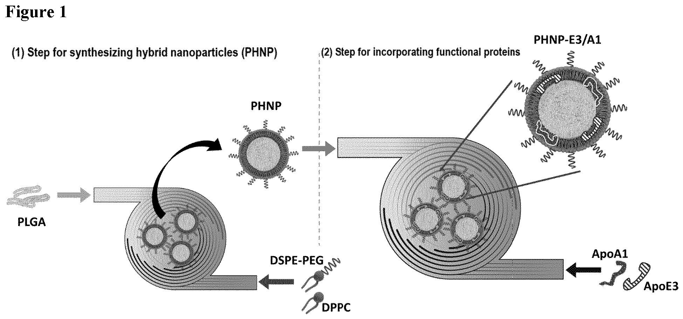

Synthesis of hybrid nanoparticles comprising apolipoproteins was performed. Specifically, step 1 is a step for synthesizing polymer-lipid hybrid nanoparticles (PHNP), step 2 is a step for incorporating apolipoproteins into hybrid nanoparticles, and the same swirling microvortex device was used in each step. A schematic diagram of the swirling microvortex device and the synthesis method used for the synthesis of the hybrid nanoparticle (PHNP) of the present invention is shown in Figure 1.

The swirling microvortex device comprises two inlets and one outlet. In order to effectively mix lipids and PLGA polymers through the formation of swirling microvortices, the diameter and height of the device were optimized and designed (see, Toth et al., Robust manufacturing of lipid-polymer nanoparticles through feedback control of parallelized swirling microvortices. 2017, Lab. Chip., 17.16: 2805-2813).

In the step for synthesizing polymer-lipid hybrid nanoparticles (PHNP), a phospholipid mixture, DPPC (dipalmitoylphosphatidylcholine) and DSPE-PEG (1,2-distearoyl-sn-glycero-3-phosphoethanolamine-N-polyethylene glycol), was injected into one of the two inlets, and PLGA (poly(lactic-co-glycolic acid)) polymer was injected into one inlet located on the opposite side to synthesize polymer-lipid hybrid nanoparticles (PHNP).

Thereafter, the synthesized polymer-lipid hybrid nanoparticles (PHNP) were injected into one inlet, and apolipoproteins were injected into one inlet located on the opposite side, and then mixed to finally synthesize hybrid nanoparticles comprising apolipoproteins.

### [Example 2]

### Synthesis of polymer-lipid hybrid nanoparticles according to mixing weight ratio of DPPC:DSPE-PEG2000

Using the swirling microvortex device, polymer-lipid hybrid nanoparticles comprising lipids (DPPC and DSPE-PEG2000) and polymers (PLGA) were synthesized as follows. DSPE-PEG2000 comprises PEG, which prevents plasma proteins in the human body from being adsorbed onto the surface of nanoparticles or removed by immune cells, and was therefore used to ensure the stability of nanoparticles in the body.

PLGA was prepared as a solution dissolved in anhydrous acetonitrile (ACN), and DPPC and DSPE-PEG2000 were prepared as anhydrous ethanol solutions. After synthesis using the swirling microvortex device, the final solution was immediately purified with deionized water (DW).

In order to produce the stable polymer-lipid hybrid nanoparticles (PHNP) of the present invention, particle optimization was performed according to the lipid mixing ratio in an anhydrous ethanol solution.

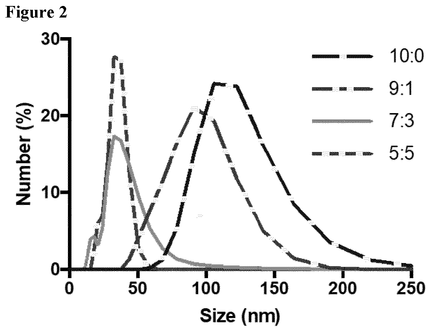

As a result of measuring the size of the nanoparticles synthesized under the conditions of synthetic mixing weight ratios of DPPC and DSPE-PEG2000 of 10:0, 9:1, 2.3:1, and 1:1, the sizes of the generated particles are shown in Figure 2. As shown in Figure 2, it was confirmed that the sizes of the particles produced under the conditions of synthetic mixing weight ratios of 10:0, 9:1, 2.3:1, and 1:1 were 130 nm and 90 nm, respectively.

In addition, the sizes of the particles produced under the conditions of synthetic mixing weight ratios of 2.3:1 and 1:1 were 30 nm, and in particular, it was confirmed that the sizes of the particles under the conditions of a synthetic mixing weight ratio of 1:1 were very homogeneous. Therefore, it can be seen that nanoparticles having small and uniform sizes are produced under the conditions of a synthetic mixing weight ratio of 2.3:1 to 1:1.

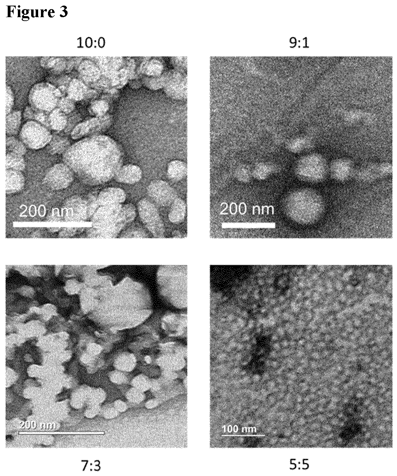

In addition, the results of observing the shape of the nanoparticles using TEM are shown in Figure 3. As shown in Figure 3, it was confirmed that when the synthetic mixing weight ratios of DPPC and DSPE-PEG2000 were 10:0 and 9:1, the particle size was not uniform, and when they were 7:3 and 5:5, the particle size was small and uniform.

### [Example 3]

### Synthesis of hybrid nanoparticles according to molecular weight of DSPE-PEG

The hybrid nanoparticles (PHNP) comprising DSPE-PEG having various PEG molecular weights were produced using the swirling microvortex device of Example 1.

PLGA was prepared as an anhydrous acetonitrile (ACN) solution, and DPPC and DSPE-PEG (550, 2000, and 5000) were prepared as an anhydrous ethanol solution, and the synthesis was performed in a swirling microvortex device with a synthetic mixing weight ratio of DPPC and DSPE-PEG of 2.3:1. The final synthesis product was purified with DW.

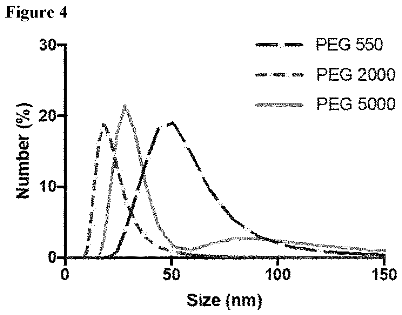

Thereafter, the sizes of the particles comprising DSPE-PEG having various PEG molecular weights were compared and are shown in Figure 4. As shown in Figure 4, in the case of hybrid nanoparticles (PHNP-PEG550) including DSPE-PEG550, it was confirmed that non-uniform particles of 50 nm were generated, and in the case of hybrid nanoparticles (PHNP-PEG5000) including DSPE-PEG5000, it was confirmed that particles of 27 nm were generated. In addition, in the case of hybrid nanoparticles (PHNP-PEG2000) including DSPE-PEG2000, it was confirmed that small and uniform particles of 18 nm were generated.

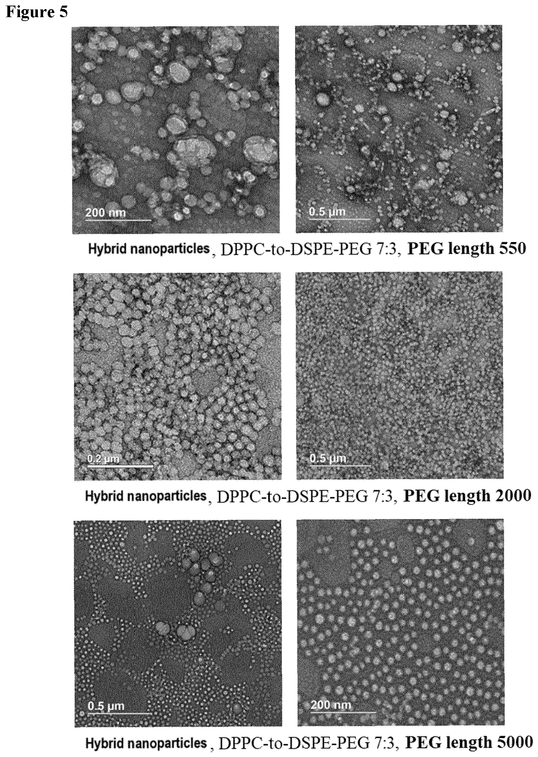

In addition, the results of observing the shape of the generated hybrid nanoparticles using TEM are shown in Figure 5. As shown in Figure 5, it can be seen that the size and shape of the generated nanoparticles vary depending on the molecular weight of PEG. It was confirmed that in the case of PHNP-PEG550, the particle size was non-uniform and had a size of 30-80 nm as measured by DLS, and in the case of PHNP-PEG2000, it had a uniform particle size of 20 nm.

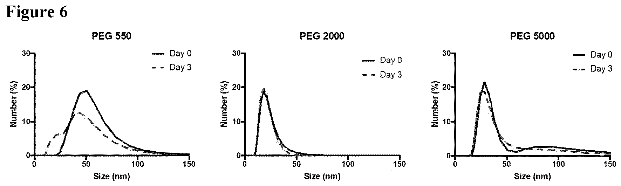

In addition, in order to confirm the stability of the generated hybrid nanoparticles, the particle size was confirmed through DLS after 3 days, and the results are shown in Figure 6. As shown in Figure 6, it was confirmed that PHNP-PEG2000 and PHNP-PEG5000 were stable without any change in particle size after 3 days.

Therefore, it can be seen that small, uniform, and stable particles can be obtained for PHNP-PEG2000 and PHNP-PEG5000.

### [Example 4]

### Synthesis of hybrid nanoparticles comprising apolipoproteins

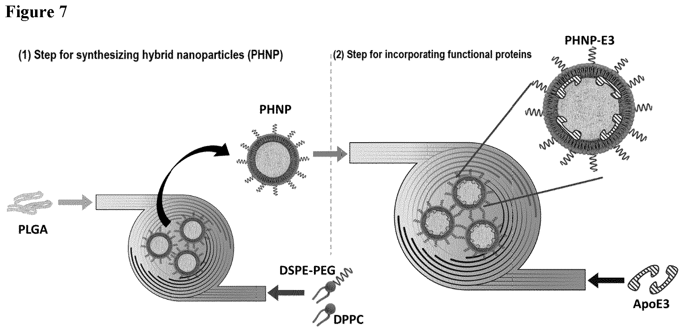

Using a swirling microvortex device, the polymer-lipid hybrid nanoparticles (PHNP-PEG2000) comprising DPPC, DSPE-PEG2000, and PLGA were produced, and then the hybrid nanoparticles (PHNP-E3) comprising apolipoprotein E3 (ApoE3) were produced, and a schematic diagram thereof is shown in Figure 7.

Specifically, PLGA was prepared as an anhydrous acetonitrile (ACN) solution, and DPPC and DSPE-PEG2000 were prepared as an anhydrous ethanol solution. After synthesis using the swirling microvortex device at a synthetic mixing ratio of DPPC:DSPE-PEG2000 of 2.3:1, the final solution was immediately purified with DW. Thereafter, the synthesized PHNP-PEG2000 and ApoE3 were injected into each inlet of the swirling microvortex device. At this time, the mixing weight ratio of PHNP-PEG2000 and ApoE3 was 4:1, and the Reynolds number was 250. The finally synthesized hybrid nanoparticles (PHNP-E3) comprising ApoE3 were purified using an Amicon filter (MWCO 50 kDa).

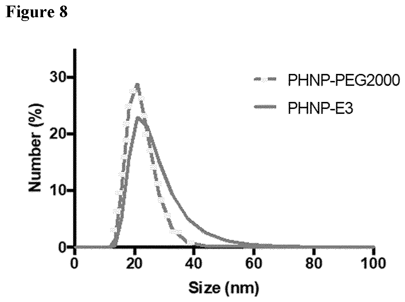

The results obtained by confirming the size of PHNP-PEG2000 without ApoE3 and PHNP-E3 comprising ApoE3 by DLS are shown in Figure 8. As shown in Figure 8, it was confirmed that PHNP-PEG2000 had a particle size of 21 nm, and PHNP-E3 had a particle size of 24 nm. In addition, as a result of confirming the amount of ApoE3 using the BCA assay during the purification process, and it was confirmed that 70% of ApoE3 was included in the final formed nanoparticles.

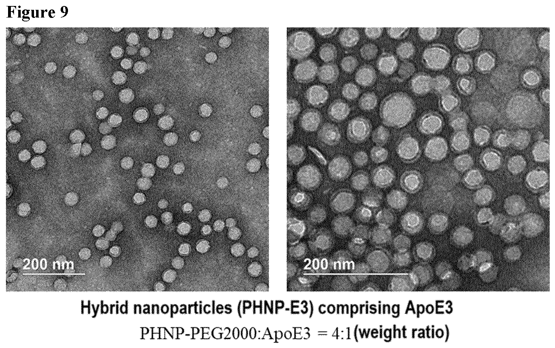

In addition, as shown in Figure 9, as a result of observing the synthesized PHNP-E3 using TEM, a core-shell structure with a core in the middle of the nanoparticles was observed.

Therefore, it can be seen that small and uniform PHNP-E3 with a core-shell is obtained through the above method.

### [Example 5]

### Synthesis of hybrid nanoparticles comprising apolipoproteins according to Reynolds flow rate

Using a swirling microvortex device, hybrid nanoparticles (PHNP-PEG2000) comprising DPPC, DSPE-PEG 2000, and PLGA were synthesized in the same manner as in Figure 7, and then ApoE3 was incorporated to finally synthesize hybrid nanoparticles (PHNP-E3).

In the same manner as in Example 4, PLGA was prepared as an anhydrous acetonitrile (ACN) solution, and DPPC and DSPE-PEG2000 were prepared as an anhydrous ethanol solution, and synthesized using a swirling microvortex device, and then the final solution was purified with DW. The synthesized hybrid nanoparticles (PHNP-PEG2000) and ApoE3 protein were injected into each inlet according to the synthesis ratio. At this time, the ratio of PHNP-PEG2000 and ApoE3 was 10:1.

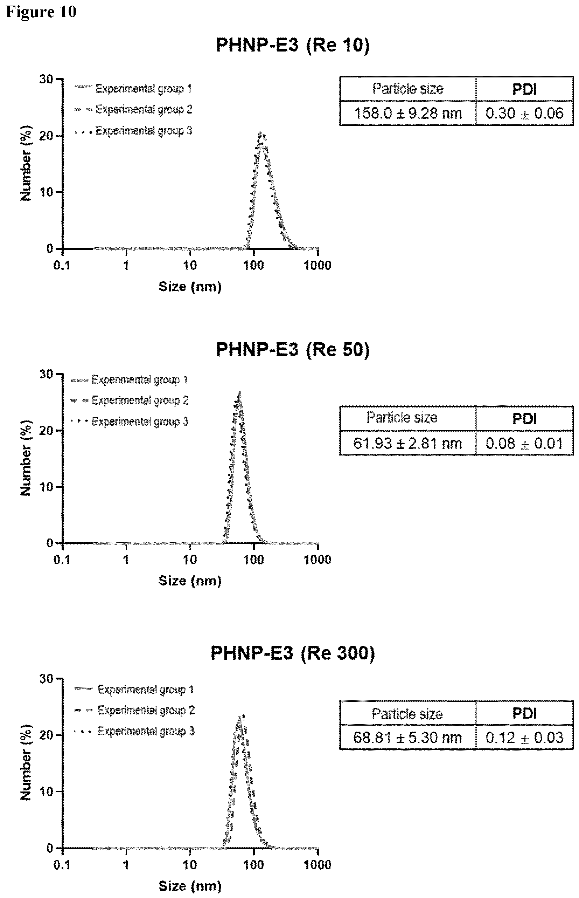

PHNP-E3 particles were synthesized at various Reynolds flow rates, and the The size of the particles generated distribution was confirmed by DLS, and the results are shown in Figure 10. As shown in Figure 10, under conditions where the Reynolds flow rate was less than 50 (Re 10), relatively large particles were measured with an average particle size of 158 nm compared to other conditions. This may be because under excessively slow flow rate conditions, normal PHNP-E3 is not synthesized, and E3 is adsorbed on the surface of PHNP-PEG2000, forming aggregates.

In addition, it was confirmed that PHNP-E3 showing a normal particle size distribution was synthesized under the conditions of the Reynolds flow rate of 300.

In the swirling microvortex device, the mixing efficiency increases in proportion to the Reynolds flow rate, but when it reaches the highly mixed regime, the mixing efficiency stops increasing and converges to the maximum value (Manon, R., et al. Optimal self-assembly of lipid nanoparticles (LNP) in a ring micromixer, Scientific reports, 12:9483 (2022)). Therefore, after reaching the highly mixed regime, there is no need to increase the Reynolds flow rate. In addition, excessive Reynolds flow rate can negatively affect the three-dimensional structure of proteins. For example, in the case of insulin, it is known that structural changes and aggregates are formed based on a shear rate of 200 s⁻¹ or a shear stress of 1000 dyne/cm² (Bekard, I., et al. The Effects of Shear Flow on Protein Structure and Function. Biopolymers, 95, 11, 733-745 (2011)).

Ultimately, the actual flow rate is 14.14 mL/min under the conditions of the Reynolds flow rate of 300. Since a flow rate condition higher than the above value can apply excessive pressure to the microvortex device and protein, it can be seen that it is preferable to have a condition where the Reynolds flow rate in the present invention is 50 to 300.

### [Example 6]

### Optimization of synthetic mixing ratio of hybrid nanoparticles comprising apolipoproteins

PHNP-E3 was produced by varying the synthetic mixing ratio of PHNP-PEG2000 and ApoE3 (20:1, 10:1, 5:1, 2:1, 1:1, 0.5:1). At this time, the amount of PHNP-PEG2000 used was 1000 µg, and the amount of ApoE3 is shown in Table 1 below.

**[Table 1]**

| | PHNP-PEG2000:ApoE3 | | | | | |
|---|---|---|---|---|---|---|
| PHNP-PEG2000:ApoE3 synthetic mixing weight ratio | 20:1 | 10:1 | 5:1 | 2:1 | 1:1 | 0.5:1 |
| ApoE3 (µg) added | 50 | 100 | 200 | 500 | 1000 | 2000 |
| ApoE3 (µg) included in PHNP-E3 | 31.4 | 55.6 | 112 | 231 | 545 | 1100 |
| Particle size (nm) | 60.3 | 64.2 | 58.5 | 52.9 | 47.6 | 27.7 |
| PDI | 0.127 | 0.029 | 0.12 | 0.172 | 0.193 | 0.215 |

In the same manner as in Example 4, PLGA was prepared as an anhydrous acetonitrile (ACN) solution, and DPPC and each DSPE-PEG2000 were prepared as an anhydrous ethanol solution, and synthesized using a swirling microvortex device, and then the final solution was purified with DW. PHNP-PEG2000 and ApoE3 were injected into each inlet at the synthetic mixing ratio according to Table 1 above. The size of the particles generated according to the synthetic mixing ratios of PHNP-PEG2000 and ApoE3 (20:1, 10:1, 5:1, 2:1, 1:1, 0.5:1) was confirmed by DLS. As shown in Table 1 above, it was confirmed that PHNP-E3 (20:1) had a particle size of 60.3 nm, PHNP-E3 (10:1) had a particle size of 64.2 nm, PHNP-E3 (5:1) had a particle size of 58.5 nm, and PHNP-E3 (2:1) had a particle size of 52.9 nm, and all of them were stable with a polydispersity index (PDI) of 0.18 or less. The closer the PDI is to 0, the more the nanoparticles exist in a uniform size, and generally, nanoparticles have a value of 0.3 to 0.4. The closer the PDI is to 0, the more the nanoparticles exist in a uniform size, and generally, nanoparticles have a value of 0.3 to 0.4.

On the other hand, it was confirmed that the nanoparticles (PHNP-E3 (1:1)) had a particle size of 47.6 nm, and PHNP-E3 (0.5:1) had a particle size of 27.7 nm, and these particles had an increased PDI of more than 0.18 compared to the above particles.

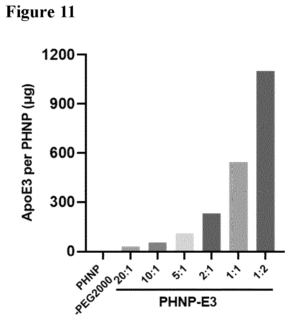

In addition, the amount of ApoE3 included in PHNP-E3 was confirmed according to the synthetic mixing weight ratio using the BCA assay, a protein quantification method. As shown in Table 1 above and Figure 11, it was confirmed that PHNP-E3 (20:1) included 31.4 µg of ApoE3, PHNP-E3 (10:1) included 55.6 µg of ApoE3, PHNP-E3 (5:1) included 112 µg of ApoE3, PHNP-E3 (2:1) included 231 µg of ApoE3, PHNP-E3 (1:1) included 545 µg of ApoE3, and PHNP-E3 (0.5:1) included 1100 µg of ApoE3. Therefore, it was confirmed that the amount of ApoE3 included in PHNP-E3 was included in a concentration-dependent manner according to the synthetic mixing weight ratio.

In addition, PHNP-PEG-2000 comprises PEG-2000 in the shell of the nanoparticle, but when synthesized by adding apolipoprotein, it was confirmed that it was stably incorporated into PHNP-PEG-2000 in proportion to the amount of apolipoprotein added. This cannot be obtained by conventional synthetic methods, and can be obtained through a process of physically pushing apolipoprotein into PHNP-PEG-2000 as inelastic collision by controlling the Reynolds flow rate using a swirling microvortex device. It can be seen that the PHNP-E3 obtained in this way has small, uniform, and stable particles.

### [Example 7]

### Method for synthesizing hybrid nanoparticles comprising apolipoprotein (ApoA1)

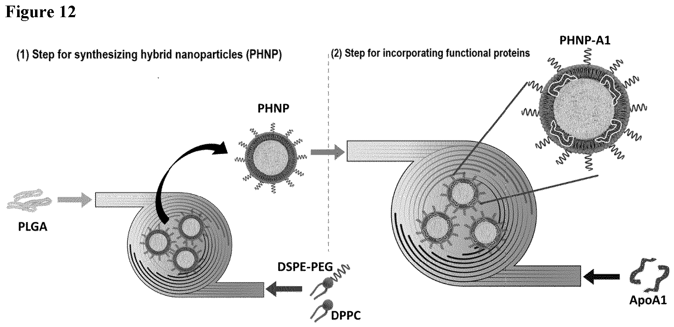

The hybrid nanoparticles (PHNP-A1) comprising ApoA1 were synthesized using a swirling microvortex device in the same manner as in Figure 12.

Specifically, PLGA was prepared as an anhydrous acetonitrile (ACN) solution, and DPPC and each DSPE-PEG2000 were prepared as an anhydrous ethanol solution. Synthesis was performed using a swirling microvortex device at a mixing ratio of DPPC:DSPE-PEG2000 of 2.3:1, and the final solution was purified with DW to produce PHNP-PEG2000.

The synthesized PHNP-PEG2000 and apolipoprotein A1 (ApoA1) were injected into each inlet of the swirling microvortex device. At this time, the Reynolds number was set to 250, and the finally synthesized hybrid nanoparticles (PHNP-A1) comprising ApoA1 were purified using an Amicon filter (MWCO 50 kDa).

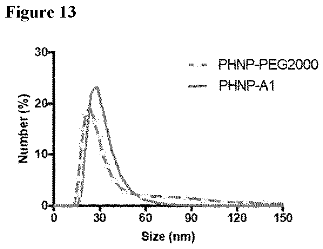

The results obtained by confirming the sizes of the hybrid nanoparticles (PHNP-PEG2000) with ApoA1 and the hybrid nanoparticles (PHNP-A1) comprising ApoA1 by DLS are shown in Figure 13. As shown in Figure 13, it was confirmed that PHNP-PEG2000 had a particle size of 21 nm, and PHNP-A1 had a particle size of 25 nm.

In addition, as a result of confirming with BCA assay to quantify ApoA1 during the purification process, it was confirmed that 26% of ApoA1 was included in PHNP-PEG2000.

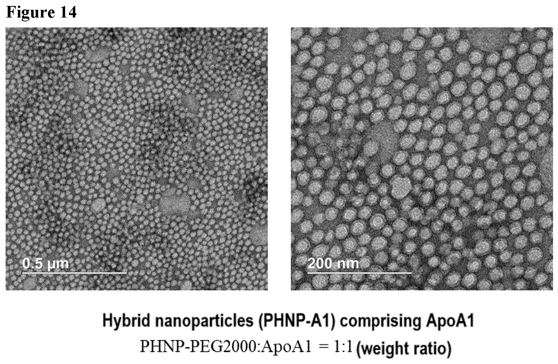

In addition, as a result of observing the shape of PHNP-A1 using TEM, it was confirmed that PHNP-A1 had a core-shell structure with a core in the center, as shown in Figure 14.

### [Example 8]

### Confirmation of intracellular delivery of hybrid nanoparticles comprising apolipoprotein (ApoA1)

The intracellular delivery of PHNP-PEG2000 and PHNP-A1 labeled with rhodamine into HAECs (P4) was observed using a confocal laser scanning microscope (CLSM).

Rhodamine corresponding to 30% of the mass of PLGA was added to the PLGA solution. After HAEC cells were treated with PHNP-PEG2000 and PHNP-A1, respectively, the cells were cultured for 24 hours, and then fixed with a 4% paraformaldehyde solution. Thereafter, the cells were stained with DAPI and then observed using a confocal laser scanning microscope.

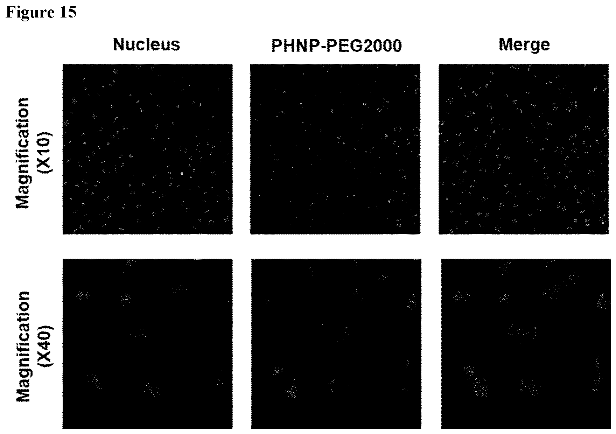

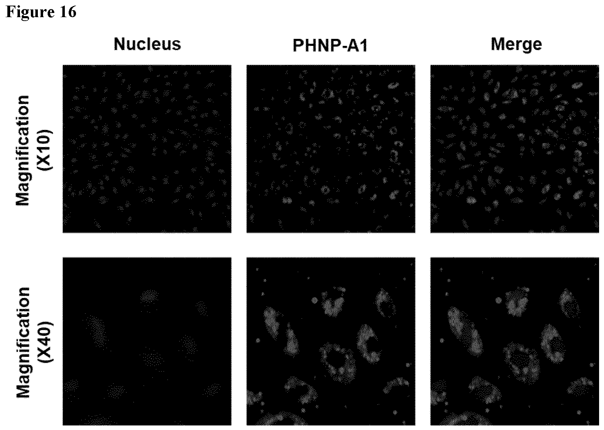

The intracellular delivery images of PHNP-PEG2000 and PHNP-A1 are shown in Figures 15 and 16. As shown in Figures 15 and 16, it was confirmed that PHNP-A1 was delivered more into HAECs cells due to its intracellular entry effect through the receptor of ApoA1 compared to PHNP-PEG2000.

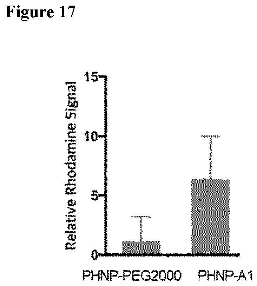

In addition, the results obtained by quantifying the fluorescence intensities of the images in Figures 15 and 16 are shown in Figure 17. As a result of comparing the fluorescence intensities, it can be seen that the intracellular delivery of PHNP-A1 into HAECs is more than 6 times higher than that of PHNP. Ultimately, it can be seen that PHNP-A1 has an excellent effect on intracellular delivery.

### [Example 9]

### Comparative evaluation of method for synthesizing hybrid comprising apolipoprotein (ApoA1)

PHNP-A1 (SMR) comprising ApoA1 was synthesized using a swirling microvortex device (SMR) in the same manner as in Example 7.

In addition, PHNP-A1 (BT) comprising ApoA1 was synthesized using a general bench-top (BT) method that does not use the microvortex device of the present invention. The bench-top method used general magnetic stirring.

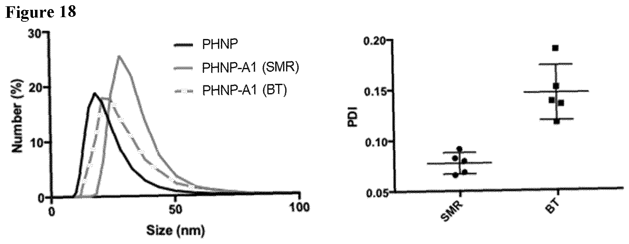

In the process of synthesizing PHNP-A1 (SMR) and PHNP-A1 (BT), the synthetic mixing weight ratio of PHNP-PEG2000 and ApoA1 was 2:1, and the results are shown in Figure 18.

As shown in Figure 18, it was confirmed that PHNP-A1 (SMR) had an increased particle size compared to PHNP-PEG2000, while PHNP-A1 (BT) had a similar particle size to PHNP-PEG2000.

In addition, the polydispersity index (PDI) results showed that PHNP-A1 (SMR) had a lower PDI value compared to PHNP-A1 (BT), and thus, it can be seen that PHNP-A1 (SMR) has a uniform particle size.

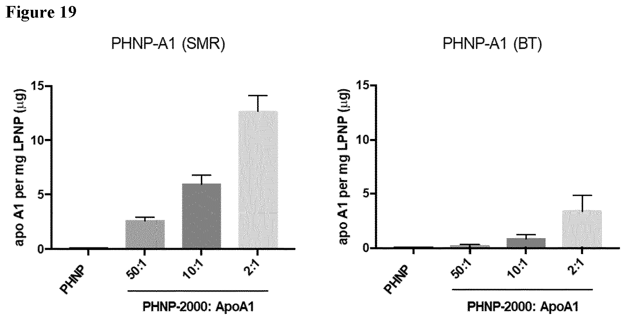

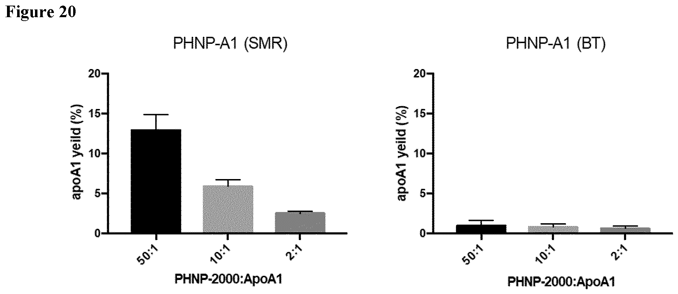

PHNP-A1 (SMR) and PHNP-A1 (BT) were synthesized at the synthetic mixing weight ratio of PHNP-2000 and ApoA1 of 50: 1, 10:1, and 2:1. The results of ApoA1 quantification using ELISA are shown as a yield amount in Figure 19 and as a yield rate in Figure 20.

As shown in Figures 19 and 20, when PHNP-A1 (SMR) and PHNP-A1 (BT) are synthesized with the same synthetic mixing weight ratio, it was confirmed that PHNP-A1 (SMR) had higher values in the yield amount and yield rate of ApoA1. Therefore, it can be seen that the synthesis method using a microvortex device is significantly excellent in incorporating apolipoprotein into nanoparticles.

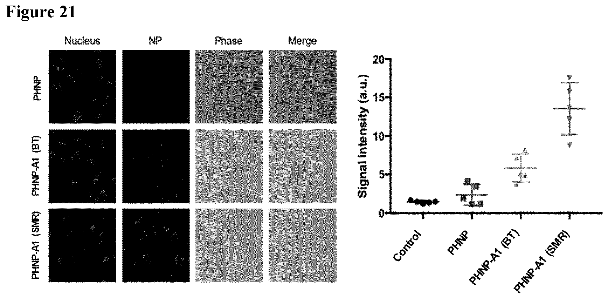

In addition, human intrahepatic biliary epithelial cells (iHBEC) were treated with PHNP-2000, PHNP-A1 (BT), and PHNP-A1 (SMR), respectively, and confocal microscopy images were taken 24 hours later, and the fluorescence intensity was quantified. As shown in Figure 21, as a result of confirmation through fluorescence imaging, it was confirmed that PHNP-A1 (SMR) was delivered to the cells the most, and it was confirmed that it was delivered more than twice as much as PHNP-A1 (BT).

As confirmed in Figures 19 and 20, it can be seen that this is because PHNP-A1 (SMR) comprises a larger amount of apolipoprotein than PHNP-A1 (BT).

Ultimately, it can be seen that the method of using a microvortex device as a synthetic method for adding proteins to nanoparticles is significantly excellent compared to the conventional synthetic method.

### [Example 10]

### Method for synthesizing hybrid nanoparticles comprising apolipoprotein (ApoE3 and ApoA1)

In the same manner as in Example 1, PHNP-PEG2000 comprising DPPC, DSPE-PEG2000, and PLGA was produced using a swirling microvortex device, and then PHNP-E3/A1 was synthesized by incorporating both ApoA1 and ApoE3.

In order to produce PHNP-E3/A1 comprising both ApoE3 and ApoA1, ApoE3 and ApoA1 were dissolved in PBS (phosphate buffered saline) at a weight ratio (ApoE3 (mg)/ApoA1 (mg)) shown in Table 2 below and prepared as a mixed aqueous solution.

**[Table 2]**

| **PHNPs** | **PHNP-E3** | **PHNP-E3/A1** | | | | **PHNP-A1** |
|---|---|---|---|---|---|---|
| ApoE3:ApoA1 (weight ratio) | - | 10:1 | 5:1 | 5:2 | 1:1 | - |
| ApoE3 (µg) added | 500 | 250 | | | | - |
| ApoA1 (µg) added | - | 25 | 50 | 100 | 250 | 500 |

PHNP-E3/A1 was synthesized by injecting a mixed aqueous solution of PHNP-PEG2000 and ApoE3/ApoA1 into each inlet of a microvortex device. At this time, the Reynolds number was set to 250. The final synthesized PHNP-E3/A1 was purified by centrifugation using an Amicon filter (MWCO 50 kDa).

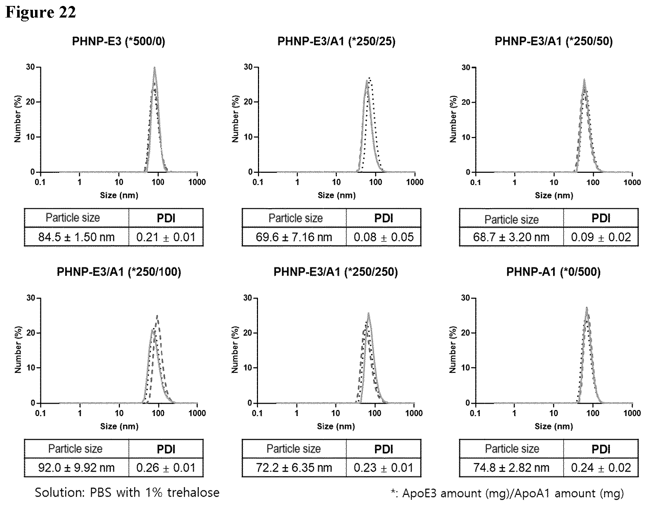

The particle size and polydispersity index (PDI) of PHNP-E3/A1 were determined by DLS in a PBS (1% trehalose) buffer solution environment. As shown in Figure 22, it was confirmed that PHNP-E3 (500/0) had a size of 85.55 ± 1.77 nm and a PDI of 0.23 ± 0.03, PHNP-E3/A1 (250/25) had a size of 70.38 ± 5.31 nm and a PDI of 0.08 ± 0.03, PHNP-E3/A1 (250/50) had a size of 68.74 ± 3.20 nm and a PDI of 0.09 ± 0.02, PHNP-E3/A1 (250/100) had a size of 96.58 ± 12.16 nm and a PDI of 0.26 ± 0.01, PHNP-E3/A1 (250/250) had a size of 73.03 ± 5.45 nm and a PDI of 0.23 ± 0.01, and PHNP-A1 (0/500) had a size of 75.55 ± 2.88 nm and a PDI of 0.26 ± 0.03. Therefore, through the results of a size of less than 100 nm and a PDI of less than 0.3, it can be seen that ApoE3 and ApoA1 are uniform and highly dispersed in the produced PHNP-E3/A1.

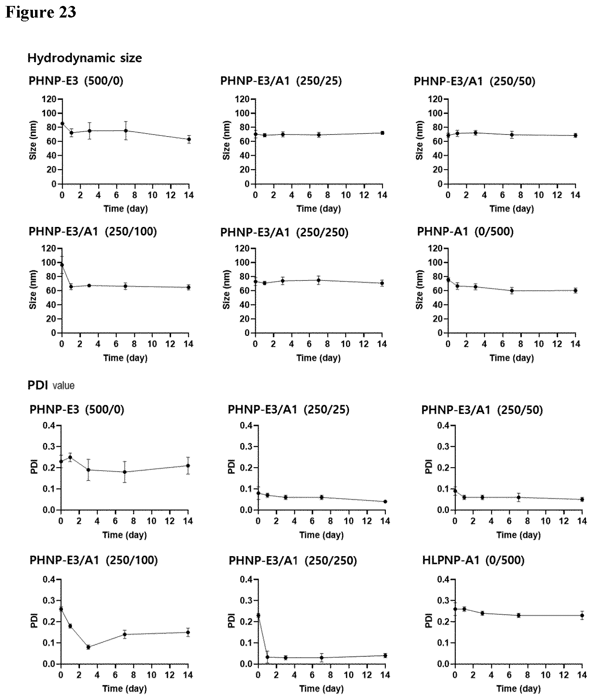

The results obtained by confirming the dispersion stability of PHNP-E3/A1 synthesized according to the synthetic mixing weight ratio of ApoE3 and ApoA1 under the conditions of 4 °C, PBS (1% trehalose) solution by DLS are shown in Figure 23. As shown in Figure 23, it was confirmed that all PHNP-E3/A1 had a size of less than 80 nm and a PDI of less than 0.3 until day 14. This means that PHNP-E3/A1 maintained high dispersion stability for 14 days without size change.

In addition, it was confirmed that ApoE3 and ApoA1 were simultaneously introduced into the nanoparticles of PHNP-PEG2000 through fluorescence resonance energy transfer (FRET). That is, in order to confirm the fluorescence intensity, Alexa Fluor 488 fluorescent molecules were attached to ApoE3, and Alexa Fluor 568 fluorescent molecules were attached to ApoA1. If Alexa Fluor 488 attached to ApoE3 emits light energy of 470 nm, which can emit fluorescence, and Alexa Fluor 568 fluorescent molecule attached to ApoA1 emits light energy of 600 nm, which is a fluorescence wavelength, then ApoE3 and ApoA1 can be said to exist simultaneously in the nanoparticle.

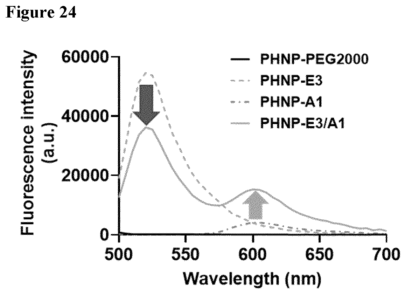

In order to confirm this, the results of the emitted fluorescence wavelength when 470 nm energy was given to PHNP-PEG2000, PHNP-E3, PHNP-A1, and PHNP-E3/A1 are shown in Figure 24. As shown in Figure 24, the control group PHNP-PEG2000 showed almost no fluorescence intensity, and PHNP-E3 showed the highest intensity at 520 nm because Alexa Fluor 488 emits fluorescence. In addition, PHNP-A1 emitted fluorescence at 600 nm, but it was confirmed that the intensity was weaker than that of PHNP-E3. In PHNP-E3/A1, it was confirmed that the fluorescence intensity of Alexa Fluor 488 attached to ApoE3 was reduced compared to PHNP-E3 (520 nm, dark gray arrow), and the fluorescence intensity of Alexa Fluor 568 attached to ApoA1 was increased compared to PHNP-A1 (600 nm, light gray arrow). The fluorescence spectrum of PHNP-E3/A1 was compared with those of PHNP-E3 and PHNP-A1, respectively. As a result, the FRET effect was demonstrated through a simultaneous decrease in the fluorescence intensity of Alexa Fluor 488 and an increase in the fluorescence of Alexa Fluor 568. Through this, it was confirmed that two types of proteins exist simultaneously in PHNP-E3/A1.

## Claims

1. A method for producing a hybrid nanoparticle comprising a protein, wherein the method comprises:
a step of injecting a hybrid nanoparticle into the first inlet and injecting a protein into the second inlet in a swirling microvortex device comprising a first inlet, a second inlet, and an outlet; and
a step of adding the protein to the hybrid nanoparticle under a swirling microvortex.

2. The method for producing a hybrid nanoparticle comprising a protein according to claim 1, wherein the Reynolds flow rate in the swirling microvortex device is 50 to 300.

3. The method for producing a hybrid nanoparticle comprising a protein according to claim 1, wherein the protein is an apolipoprotein or a polymer having amphiphilic properties.

4. The method for producing a hybrid nanoparticle comprising a protein according to claim 1, wherein the apolipoprotein is at least one selected from the group consisting of apolipoproteins A1, A2, E2, E3, J, and M.

5. The method for producing a hybrid nanoparticle comprising a protein according to claim 3, wherein the synthetic mixing weight ratio of the hybrid nanoparticle and the apolipoprotein is 20: 1 to 0.5:1.

6. The method for producing a hybrid nanoparticle comprising a protein according to claim 3, wherein the synthetic mixing weight ratio of the hybrid nanoparticle and the apolipoprotein is 20: 1 to 2:1.

7. The method for producing a hybrid nanoparticle comprising a protein according to claim 1, wherein the method further comprises a step of recovering a nanoparticle comprising a protein from the outlet.

8. The method for producing a hybrid nanoparticle comprising a protein according to claim 1, wherein the hybrid nanoparticle is produced by a production method comprising: a step of injecting a phospholipid into the first inlet and injecting a polymer into the second inlet in the swirling microvortex device; and a step of mixing the phospholipid and the polymer under a swirling microvortex.

9. The method for producing a hybrid nanoparticle comprising a protein according to claim 8, wherein the phospholipid is at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-distearoyl-sn-glycero-3-phosphocholine (DAPC), 1,2-diarachidoyl-sn-glycero-3-phosphocholine (DBPC), 1,2-diicosanoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), distearoylphosphatidylethanolamine-polyethylene glycol (DSPE-PEG), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-aminopropyl)amino]butylcarboxamido)ethyl]-3,4-di[oleyloxy]-benzamide) (VL-5), dioctadecylamidoglycylspermine 4-trifluoroacetic acid (DOGS), 3β-[N-(N',N'-dimethylaminoethane)-carbamoyl]cholesterol (DC-Chol), 1,2-di-O-octadecenyl-3-trimethylammonium propane (DOTMA), 1,2-dioleyl-3-trimethylammonium-propane (DOTAP), (1,2-dioleyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), D-Lin-MC3-DMA (MC3, DLin-MC3-DMA), DLin-KC2-DMA, and DLin-DMA.

10. The method for producing a hybrid nanoparticle comprising a protein according to claim 9, wherein the phospholipid is DPPC and DSPE-PEG.

11. The method for producing a hybrid nanoparticle comprising a protein according to claim 10, wherein the molecular weight of PEG in the DSPE-PEG is 2000 to 5000.

12. The method for producing a hybrid nanoparticle comprising a protein according to claim 10, wherein the synthetic mixing weight ratio of DPPC and DSPE-PEG is 2.3:1 to 1:1.

13. The method for producing a hybrid nanoparticle comprising a protein according to claim 8, wherein the polymer is PLGA (poly(lactic-co-glycolic acid)).

14. The method for producing a hybrid nanoparticle comprising a protein according to claim 8, wherein the hybrid nanoparticle is a polymer-lipid hybrid nanoparticle (PHNP).

15. A hybrid nanoparticle comprising a protein, wherein the hybrid nanoparticle is produced by the production method according to any one of claims 1 to 14.

16. A method for producing a hybrid nanoparticle comprising a protein, wherein the method comprises:
a first step for producing a hybrid nanoparticle, wherein the first step comprises a step of injecting a phospholipid into the first inlet and injecting a polymer into the second inlet in a swirling microvortex device comprising a first inlet, a second inlet, and an outlet; and a step of mixing the phospholipid and the polymer under a swirling microvortex;
a second step for producing a hybrid nanoparticle comprising a protein, wherein the second step comprises a step of injecting the obtained hybrid nanoparticle into the first inlet and injecting a protein into the second inlet.

17. The method for producing a hybrid nanoparticle comprising a protein according to claim 16, wherein the protein is an apolipoprotein or a polymer having amphiphilic properties.

18. The method for producing a hybrid nanoparticle comprising a protein according to claim 16, wherein the synthetic mixing weight ratio of the hybrid nanoparticle and the apolipoprotein is 20:1 to 2:1.

19. The method for producing a hybrid nanoparticle comprising a protein according to claim 16, wherein the phospholipid is at least one selected from the group consisting of 1,2-dioleoyl-sn-glycero-3-phosphatidylcholine (DOPC), egg phosphatidylcholine (EPC), dilauroylphosphatidylcholine (DLPC), 1,2-dimyristoyl-sn-glycero-3-phosphocholine (DMPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), 1,2-distearoyl-sn-glycero-3-phosphocholine (DAPC), 1,2-diarachidoyl-sn-glycero-3-phosphocholine (DBPC), 1,2-diicosanoyl-sn-glycero-3-phosphocholine (DEPC), palmitoyloleoylphosphatidylcholine (POPC), lysophosphatidylcholine, dilinoleoylphosphatidylcholine, distearoylphosphatidylethanolamine (DSPE), distearoylphosphatidylethanolamine-polyethylene glycol (DSPE-PEG), dimyristoylphosphatidylethanolamine (DMPE), dipalmitoylphosphatidylethanolamine (DPPE), palmitoyloleoylphosphatidylethanolamine (POPE), lysophosphatidylethanolamine, N1-[2-((1S)-1-[(3-aminopropyl)amino]-4-[di(3-aminopropyl)amino]butylcarboxamido)ethyl]-3,4-di[oleyloxy]-benzamide) (VL-5), dioctadecylamidoglycylspermine 4-trifluoroacetic acid (DOGS), 3β-[N-(N',N'-dimethylaminoethane)-carbamoyl]cholesterol (DC-Chol), 1,2-di-O-octadecenyl-3-trimethylammonium propane (DOTMA), 1,2-dioleyl-3-trimethylammonium-propane (DOTAP), (1,2-dioleyloxypropyl)-3-dimethylhydroxyethyl ammonium bromide (DORIE), 1,2-dimyristyloxy-propyl-3-dimethyl-hydroxyethyl ammonium bromide (DMRIE), 2,3-dioleyloxy-N-[2-(sperminecarboxamido)ethyl]-N,N-dimethyl-1-propanaminium trifluoroacetate (DOSPA), N-(3-aminopropyl)-N,N-dimethyl-2,3-bis(dodecyloxy)-1-propaneammonium bromide (GAP-DLRIE), N-t-butyl-N'-tetradecyl-3-tetradecylaminopropionamidine (diC14-amidine), ethylphosphocholine (Ethyl PC), dimethyldioctadecylammonium bromide (DDAB), N4-cholesteryl-spermine (GL67), 1,2-dioleyloxy-3-dimethylaminopropane (DODMA), D-Lin-MC3-DMA (MC3, DLin-MC3-DMA), DLin-KC2-DMA, and DLin-DMA.

20. The method for producing a hybrid nanoparticle comprising a protein according to claim 16, wherein the polymer is PLGA (poly(lactic-co-glycolic acid)).

21. The method for producing a hybrid nanoparticle comprising a protein according to claim 16, wherein the hybrid nanoparticle is a polymer-lipid hybrid nanoparticle (PHNP).
